# EUROPEAN PATENT APPLICATION

(11) **EP 2 284 225 A1**
(43) Date of publication of application: **16.02.2011**
(21) Application number: 09733371.0
(22) Date of filing: 16.04.2009
(51) Int. Cl.: C09C 3/12, C09C 1/40

(54) **ORGANIC-MATERIAL-COATED SYNTHETIC MICA POWDER, PRODUCTION METHOD THEREOF AND COSMETIC WHEREIN SAME IS USED**

(30) Priority: 17.04.2008 JP 2008107888
(71) Applicant: Topy Kogyo Kabushiki Kaisha, Tokyo 141-8634 (JP)
(72) Inventor: ABIKO, Masahiro, Tokyo 141-8634 (JP); NAGAHAMA, Hiroyuki, Tokyo 141-8634 (JP); OHTA, Shun-ichi, Tokyo 141-8634 (JP)
(74) Representative: Henkel, Feiler & Hänzel
(86) International application number: PCT/JP2009/057672
(87) International publication number: WO 2009/128511

(57) **Abstract**

The present invention provides an organic-coated synthetic mica powder excellent in feeling in use and water-repellent property by an organic coating layer from an organic surface treatment agent, which is firmly and chemically bound to the surface of the synthetic mica powder, and further provides, when an organosilicone having Si-H groups is used as the organic surface treatment agent, an organic-coated synthetic mica powder having very little hydrogen evolution due to residual Si-H groups. The organic-coated synthetic mica powder can be obtained by a producing method comprising a pretreatment process and a coating process, said pretreatment process comprising: contacting synthetic mica powder and an ammonium ion in water; carrying out solid-liquid separation to remove the liquid phase; and drying the solid phase at 200 °C or lower to volatilize NH₃, and said coating process comprising: attaching an organic surface treatment agent on the surface of the pretreated synthetic mica powder; and baking the organic surface treatment agent-attached synthetic mica powder to form an organic coating layer on the surface of the synthetic mica powder.

## Description

### RELATED APPLICATIONS

This application claims the priority of Japanese Patent Application No. 2008-107888 filed on April 17, 2008, which is incorporated herein by reference.

### FIELD OF THE INVENTION

The present invention relates to an organic-coated synthetic mica powder, wherein an organic coating layer, which is formed by baking an organic surface treatment agent to the surface of the synthetic mica powder, is chemically and firmly bound to the surface and the production method thereof. In particular, the present invention relates to the improvement of the feeling in use and the water-repellent property of organic-coated synthetic mica powder, and furthermore, relates to the decrease of residual Si-H groups when an organic surface treatment agent having Si-H groups is used.

### BACKGROUND OF THE INVENTION

Because mica powder consists of plate-like particles, it exerts good spreadability and high adhesion property on the skin. Therefore, a large amount of mica powder has been blended, as one of extender pigments, in makeup cosmetics such as foundations.

At present, mica can be artificially synthesized. For the synthesis of synthetic mica containing OH like in the crystal structure of natural mica, it is necessary to melt a mixture of raw materials under pressure. On the other hand, fluormica in which OH in the natural mica crystal is replaced by fluorine can be synthesized by melting under normal pressure. Therefore, industrially synthesized mica is mostly fluormica, and synthetic mica normally means such fluormica. For example, synthetic potassium phlogopite being KMg₃(AlSi₃O₁₀)F₂ in the theoretical formula, which is often blended in cosmetics among synthetic micas, is synthetic fluorphlogopite that has a structure in which OH of the natural phlogopite being KMg₃(AlSi₃O₁₀)(OH)₂ in the theoretical formula is replaced by F.

The synthetic mica does not contain impurities compared with natural mica, and therefore, has characteristics in that the degree of whiteness is higher and the color dullness is lower than natural mica. At present, the usage standard of the synthetic mica in the field of cosmetics is that the elution of fluoride ions is 20 ppm or less by the elution test in hot water carried out at 100°C for 1 hour. This is based on the concern about the influence of eluted fluoride ions to the safety and quality of the composition. Thus, when the synthetic mica is used in cosmetic application, the heating at 600 to 1350 °C is carried out to suppress the elution of fluoride ions from the synthetic mica (Patent Literature 1).

However, when the synthetic mica powder is heated at such a high temperature to decrease the fluoride ion-elution amount, its surface activity will also decrease. One of the causes is considered to be that the recrystallization or amorphization takes place on the surface of the synthetic mica and the number of OH groups that has been adsorbed on the surface decreases. Therefore, the reactivity of synthetic mica powder with an organic surface treatment agent is low compared with natural mica, and it has been difficult to provide a satisfactory water-repellent property to the synthetic mica powder.

For example, when the powder such as pigment is blended in makeup cosmetics such as foundations, the water-repellent property is provided to the powder in order to preventing wetting against sebum and sweat, namely to preventing make-up deterioration, by attaching a hydrophobic organic surface treatment agent such as an organosilicone or a silane coupling agent on the powder surface followed by baking to carry out the crosslinking polymerization of the organic surface treatment agent and its binding to the powder surface.

However, when such coating treatment with an organic surface treatment agent is carried out on the above-described synthetic mica powder with low surface activity, the binding reaction of the organic surface treatment agent to the powder surface is difficult to take place, and thus the satisfactory water-repellent property cannot be obtained. Even when the baking temperature is increased or the baking time is lengthened to promote the binding reaction between the surface of the synthetic mica powder and the organic surface treatment agent, the crosslinking polymerization reaction of the organic surface treatment agent to each other is promoted to decrease the amount of organochains in the coating layer. Thus, only the feeling in use is lowered, and the satisfactory water-repellent property cannot be obtained.

As a representative organic surface treatment agent, there is an organosilicone that has Si-H groups (organohydrogenpolysiloxane). When an organosilicone having Si-H groups is used to be baked, it is considered that an organic coating layer that is chemically bound to the powder surface is formed as follows: the organosilicone is firmly fixed by chemical bonding formed by the reaction of the Si-H groups with the OH groups, which have been adsorbed on the inorganic powder surface; and the organosilicone is crosslinked and polymerized to each other by the reaction among Si-H groups themselves, the reaction between Si-H groups and organic silyl groups such as Si-CH₃ groups, and the reaction among Si-CH₃ groups themselves.

When an organosilicone having Si-H groups is used, the water-repellent property may be provided to the above-described synthetic mica powder.
However, even when the water-repellent property could be provided, hydrogen gas was generated with time because of residual Si-H groups in the coating layer. Thus, the problems were generated in the product stability such as the expansion of product containers and the deterioration of products. If the high-temperature and/or prolonged baking treatment was carried out to decrease the residual Si-H groups, the amount of organochains in the coating layer decreased; thus, there were problems in that the feeling in use and the water-repellent property were lowered.

In Patent Literature 2, the heating at 260 to 500 °C is proposed because at the heating at about 200 °C in air can not cause Si-H groups to disappear completely, while the heating at 500 °C or higher converts them to silica owing to the burning of silicone, resulting in lowered hydrophobicity. By a baking treatment in such a temperature range, silicone-coated mica or phlogopite having hydrogen evolution amount of less than 0.1 mL/g and the contact angle with water of 110 degrees or higher is obtained. However, in Patent Literature 2, no examples are listed for the silicone-coated synthetic mica.

As described above, the surface activity of synthetic mica powder is lower than that of natural mica. Therefore, even when the baking treatment is carried out at 260 to 500 °C as described in Patent Literature 2, it is difficult to suppress the hydrogen evolution amount to be 0.1 mL/g or lower. Even if the suppression could be achieved, the amount of organochains in the coating layer decreases by the promotion of crosslinking polymerization reaction due to excess baking: thus, there are problems in that a rough and stiff feeling in use is generated and the water-repellent property becomes unsatisfactory. In addition, from the viewpoint of the production cost, baking at the lowest temperature possible and for the shortest time possible is desired.

On the other hand, in Patent Literature 3, the coated powder wherein the coating layer, consisting of a water- and oil-repellent agent and a reactive auxiliary agent, is baked on an activated inorganic powder base is disclosed. In addition, it is disclosed that the surface activation of inorganic powder is possible by heating at 100 to 1000 °C.
As described above, however, the activation of synthetic mica powder cannot be achieved by heat treatment, and the surface activity is rather lowered.

In addition, it is also described in Patent Literature 3 that the reaction with the water- and oil-repellent agent and the reactive auxiliary agent can take place more easily by etching the heat-activated inorganic powder base with an alkali or an acid or by the introduction of a functional group, as necessary.
However, it has been clarified by the investigation of the present inventors that when the synthetic mica powder is treated with an alkali such as KOH, the feeling in use and the water-repellent property after baking with an organic surface treatment agent significantly decrease. It has been also clarified that when the treatment is carried out with an acid such as citric acid, the elution of fluoride ions from the synthetic mica powder increases significantly, which results in deterioration of the quality of the synthetic mica powder.

### PRIOR ART

### PATENT LITERATURE

Patent Literature 1: Japanese Examined Patent Publication No. H07-115858
Patent Literature 2: Japanese Unexamined Patent Publication No. 2001-262004
Patent Literature 3: Japanese Patent No. 2597492

### SUMMARY OF THE INVENTION

### PROBLEM TO BE SOLVED BY THE INVENTION

The present invention was made in view of the above-described background art, and the object is to provide an organic-coated synthetic mica powder excellent in feeling in use and water-repellent property by an organic coating layer from an organic surface treatment agent, which is firmly and chemically bound to the surface of the synthetic mica powder. Another object is to provide, when an organosilicone having Si-H groups is used as the organic surface treatment agent, an organic-coated synthetic mica powder having very little hydrogen evolution due to residual Si-H groups as well as excellent feeling in use and the water-repellent property.

### MEANS TO SOLVE THE PROBLEM

The present inventors have diligently studied to solve the above problems. As a result, the present inventors have found that an organic-coated synthetic mica powder having a soft and very good feeling in use and a high water-repellent property can be obtained if the surface of the synthetic mica powder is pretreated with ammonium ions. This is because the surface activity is improved by the pretreatment and the surface of the synthetic mica powder is easily reacted with an organic surface treatment agent at a relatively low temperature. In addition, the present inventors have found that when an organosilicone having Si-H groups is used, an organic-coated synthetic mica powder having a soft and very good feeling in use and a high water-repellent property, without the concern of hydrogen evolution, can be obtained, thus leading to completion of the present invention.

That is, the method for producing organic-coated synthetic mica powder of the present invention comprises a pretreatment process and a coating process,
said pretreatment process comprising:
contacting synthetic mica powder and an ammonium ion in water;
carrying out solid-liquid separation to remove the liquid phase; and
drying the solid phase at 200 °C or lower to volatilize NH₃, and
said coating process comprising:
attaching an organic surface treatment agent on the surface of the pretreated synthetic mica powder; and
baking the organic surface treatment agent-attached synthetic mica powder to form an organic coating layer on the surface of the synthetic mica powder.

In addition, the present invention provides the method, wherein a supply source of the ammonium ion is NH₃ or a water-soluble ammonium salt.
In addition, the present invention provides any of the methods, wherein the used ammonium ion is 1 to 30 mass % in nitrogen with respect to the synthetic mica powder.
In addition, the present invention provides any of the methods, wherein the organic surface treatment agent is an organosilicone, a silane coupling agent, a titanate coupling agent, an aluminate coupling agent, or a perfluoroalkylalkoxysilane.
In addition, the present invention provides any of the methods, wherein the baking temperature is 100 to 260 °C.

In addition, the present invention provides any of the methods, wherein the organic surface treatment agent is an organosilicone and the baking temperature is 170 to 260 °C.
In addition, the present invention provides any of the methods, wherein the organic surface treatment agent contains an organosilicone having Si-H groups.
In addition, the present invention provides any of the methods, wherein the mole ratio of Si(R)₂O unit: SiH(R)O unit, wherein respective Rs are either identical or different monovalent hydrocarbon groups, in the organic surface treatment agent is within the range of 1:0.05 to 1:0.30.
In addition, the present invention provides the method, wherein R is a methyl group.

In addition, the present invention provides any of the methods, wherein
the synthetic mica powder to be pretreated has the fluoride ion-elution amount of 20 ppm or less by an elution test in hot water at 100 °C for 1 hour and has the t-butanol decomposition rate of less than 20% at 250 °C, and
the pretreated synthetic mica powder has the fluoride ion-elution amount of 20 ppm or less in the hot water elution test at 100 °C for 1 hour and has the t-butanol decomposition rate of 20% or higher at 250 °C.
In addition, the present invention provides any of the methods, wherein 20% or higher of interlayer cations, which has been present on the surface of the synthetic mica powder, are removed in the pretreatment process.

The present invention provides an organic-coated synthetic mica powder, said organic-coated synthetic mica powder being obtained by any of the methods described above , wherein the carbon amount in the organic-coated synthetic mica powder is 40 to 60 mass % of the carbon amount in the organic surface treatment agent-attached synthetic mica powder before baking.
In addition, the present invention provides the organic-coated synthetic mica powder, wherein an organosilicone having Si-H groups is used as the organic surface treatment agent, and the hydrogen evolution amount of the organic-coated synthetic mica powder is less than 0.1 mL/g.

The present invention provides an organic-coated synthetic mica powder, said organic-coated synthetic mica powder being obtained by attaching an organic surface treatment agent on the surface of synthetic mica powder followed by baking, wherein the carbon amount in the organic-coated synthetic mica powder is 40 to 60 mass % of the carbon amount in the organic surface treatment agent-attached synthetic mica powder before baking.
In addition, the present invention provides the organic-coated synthetic mica powder, wherein the organic surface treatment agent is an organosilicone, a silane coupling agent, a titanate coupling agent, an aluminate coupling agent, or a perfluoroalkylalkoxysilane.

In addition, the present invention provides any of the organic-coated synthetic mica powder, wherein the organic surface treatment agent contains an organosilicone having Si-H groups, and the hydrogen generation amount of the organic-coated synthetic mica powder is less than 0.1 mL/g.
In addition, the present invention provides the organic-coated synthetic mica powder, wherein the mole ratio of Si(R)₂O unit: SiH(R)O unit, wherein respective Rs are either identical or different monovalent hydrocarbon groups, in the organic surface treatment agent is whithin the range of 1:0.05 to 1:0.30.
In addition, the present invention provides the organic-coated synthetic mica powder, wherein R is a methyl group.

In addition, the present invention provides any of the organic-coated synthetic mica powder, wherein the fluoride ion-elution amount thereof by an elution test in hot water at 100 °C for 1 hour is 20 ppm or less.
The cosmetic composition of the present invention used any of the organic-coated synthetic mica powder.

In the synthetic mica powder to be coated with an organic surface treatment agent of the present invention, the fluoride ion elution amount thereof by an elution test in hot water at 100 °C for 1 hour is 20 ppm or less, and the t-butanol decomposition rate thereof at 250 °C is 20% or higher.
In addition, the present invention provides the synthetic mica powder to be coated with an organic surface treatment agent, wherein 20% or higher of interlayer cations, which has been present on the surface of the synthetic mica powder are removed.

### EFFECT OF THE INVENTION

According to the present invention, the synthetic mica powder is pretreated with ammonium ions to activate the surface of the synthetic mica powder. Therefore, the organic surface treatment agent easily reacts with the surface at a relatively low temperature. Thus, the coating layer is firmly bound to the powder surface even without excess baking, and a relatively large number of organochains remain in the coating layer. As a result, the organic-coated synthetic mica powder having a soft and very good feeling in use and a high water-repellent property can be obtained. When the organic surface treatment agent is an organosilicone having Si-H groups, a coating layer wherein a relatively large number of organochains remain can be firmly formed while the hydrogen evolution amount due to residual Si-H groups is lowered to less than 0.1 mL/g. Therefore, the organic-coated synthetic mica powder without the problem that hydrogen is evolved over time due to residual Si-H groups and having a soft and very good feeling in use and the high water-repellent property can be obtained. In addition, the fluoride ion-elution amount does not increase by the pretreatment or the coating treatment of the present invention. Therefore, a synthetic mica powder, to be coated with an organic surface treatment agent, having the fluoride ion- elution amount of 20 ppm or less can be obtained. Thus, the organosilicone-coated synthetic mica powder of the present invention can be suitably used especially in cosmetic compositions.

### BRIEF DESCRIPTION OF THE DRAWINGS

Fig. 1 shows the relationship between the baking time and (a) the hydrogen evolution amount or (b) the residual carbon ratio when the baking treatment with an organic surface treatment agent having Si-H groups is carried out for the synthetic mica powder pretreated with ammonia or KOH or for the synthetic mica powder without pretreatment.
Fig. 2 shows the relationships among the baking temperature, the baking time, and (a) the hydrogen evolution amount or (b) the residual carbon ratio when the baking treatment with an organic surface treatment agent having Si-H groups is carried out for the synthetic mica powder pretreated with ammonium carbonate.

Fig. 3 shows the relationship between the drying temperature and the residual carbon ratio for the obtained organic-coated synthetic mica powder when the synthetic mica powder was pretreated with ammonia.
Fig. 4 shows the effects to the residual carbon ratio and the hydrogen evolution amount when natural inorganic powders were pretreated with ammonia.
Fig. 5 shows the residual carbon ratio and the hydrogen evolution amount when the removal of the liquid phase by solid-liquid separation was not carried out in the pretreatment process.

### MODE FOR CARRYING OUT THE INVENTION

The organic-coated synthetic mica powder of the present invention is the synthetic mica powder having an organic coating layer, which is formed by baking an organic surface treatment agent, on the surface of the synthetic mica powder. The organic coating layer is firmly bonded chemically to the surface of the synthetic mica powder, and a relatively large number of organochains remain in the coating layer. Therefore, the organic-coated synthetic mica powder exerts roughness-free soft feeling in use as well as the high water-repellent property.
Such an organic-coated synthetic mica powder can be obtained, as described later, by the pretreatment of synthetic mica powder with ammonium ions and the subsequent coating treatment with an organic surface treatment agent.
The carbon amount in the organic-coated synthetic mica powder of the present invention is preferably 40 to 60 mass % of the carbon amount in the organic surface treatment agent-attached synthetic mica powder before baking, and more preferably 45 to 60 mass %.
If the carbon amount is less than 40%, the roughness of the powder is felt and the water-repellent property decreases. If the carbon amount is more than 60%, even when the pretreatment by ammonium ions is carried out, the binding between the synthetic mica and the organic surface treatment agent is not satisfactory and the water-repellent property may be inferior.

In addition, when the organic surface treatment agent is an organosilicone having Si-H groups, it is preferable to have the following characteristics:
(a) the carbon amount in the organic-coated synthetic mica powder is preferably 40 to 60 mass % of the carbon amount in the organic surface treatment agent-attached synthetic mica powder before baking, and more preferably 45 to 60 mass %; and
(b) the hydrogen evolution amount of the organic-coated synthetic mica powder is less than 0.1 mL/g.
If the carbon amount is less than 40%, the roughness of the powder is felt and the water-repellent property decreases. If the carbon amount is more than 60%, the hydrogen evolution amount may become 0.1 mL/g or higher even when the pretreatment with ammonium ions is carried out.

The organic-coated synthetic mica powder of the present invention can be obtained, as described in the following, by the pretreatment of the synthetic mica powder with ammonium ions followed by the coating treatment with an organic surface treatment agent.

### <Pretreatment Process>

After the synthetic mica powder is contacted with ammonium ions in water, the liquid phase is removed by solid-liquid separation, and the obtained solid phase is dried at 200 °C or lower to volatilize NH₃.
The surface activity of the synthetic mica powder is improved by such a pretreatment process. For example, when the t-butanol decomposition rate is used as the indicator of surface activity, the t-butanol decomposition rate for the synthetic mica powder, which is normally used as a cosmetic raw material, is usually 5% or lower, and 10% at the most. On the other hand, when the synthetic mica powder is pretreated with ammonium ions, the t-butanol decomposition rate can be increased to 20% or higher, and even 35% or higher can be achieved.

In the following, the explanation is made with reference to a typical example. Initially, the synthetic mica powder is added into water to prepare a slurry. The concentration of the synthetic mica powder in the slurry is not limited in particular; however, it is preferably 1 to 30 mass % in normal cases.
Subsequently, a supply source of ammonium ions is added to the slurry. As the supply source of ammonium ions, the material that can volatilize from the powder surface at 200 °C or lower is preferable. Examples thereof include water-soluble ammonium salts such as ammonium carbonate, ammonium hydrogencarbonate, ammonium formate, and ammonium acetate as well as ammonia. The preferable material is ammonia, ammonium carbonate, or ammonium hydrogencarbonate. In the present invention, one or more supply sources of ammonium ions can be used.

The amount of the supply source of ammonium ions is used preferably 1 to 30 mass % in the amount of nitrogen, with respect to the synthetic mica powder, and more preferably 1 to 20 mass %. If the amount is too small, the effect of the present invention cannot sufficiently be obtained. On the other hand, even when an excess amount is used, an increase in the effect corresponding to the increased amount cannot be expected, and the volatilization of the ammonium salt may take a longer time.
An aqueous solution of the supply source of ammonium ions may be prepared to be added. Furthermore, the contact of the synthetic mica powder with ammonium ions may be carried out by adding the synthetic mica powder to an aqueous solution of the supply source of ammonium ions to form a slurry.

The contact between the synthetic mica powder and ammonium ions is normally sufficient if the contact is carried out at room temperature for about 1 to 10 minutes. By the contact with ammonium ions, at least a portion of the interlayer cations on the surface of the synthetic mica powder are considered to be replaced with ammonium ions.

Subsequently, the liquid phase is removed by solid-liquid separation, and the obtained solid phase is dried at 200 °C or lower. The method of solid-liquid separation is not limited in particular, and publicly known methods such as filtration and centrifugal separation can be adopted. By removing the liquid phase, the interlayer cations replaced with ammonium ions and excess ammonium ions are removed to the outside of the system. On this occasion, it is preferable, from the standpoint of reactivity improvement, that 20% or higher of interlayer cations on the surface of the synthetic mica powder are removed.
The ammonium ions that have replaced and adsorbed on the surface of the synthetic mica powder are removed as ammonia by drying to form reactive sites (OH groups) on the surface of the synthetic mica powder. On this occasion, if the drying temperature is too high, the reactive sites formed on the surface of the synthetic mica disappear, and the effect of the present invention cannot be obtained. Therefore, the drying temperature is preferably 200°C or lower, and more preferably 180 °C or lower. As the drying method, reduced-pressure drying or freeze-drying can also be used.

If ammonium ions remain on the surface of the synthetic mica, the crosslinking polymerization reaction of the organic surface treatment agent to each other by baking the organic surface treatment agent is promoted. As a result, the residual carbon ratio decreases, and the water-repellent property and the feeling in use become unsatisfactory. Therefore, it is important, in the pretreatment process, to completely remove ammonium ions on the surface of the synthetic mica.

The synthetic mica powder that is used as a raw material in the present invention can be any publicly known synthetic mica powder. The synthetic mica powder obtained by any preparation method such as the melting method, hydrothermal method, or the intersolid reaction method can be used. Normally, the synthetic mica powder of good crystal quality can be obtained by mixing compounds containing potassium, sodium, magnesium, aluminum, silicon, fluorine, etc. at a fixed ratio, melting the mixture, crystallizing out, cooling, mechanically crushing, heat-treating, washing, and drying.

Synthetic mica can be represented by the following formula:

X_{1/3-1} Y₂₋₃(Z₄ O₁₀)F₂

wherein X represents one or more ions selected from the group consisting of Na⁺, K⁺, Li⁺, Ca²⁺, Rb⁺, and Sr²⁺;
Y represents one or more ions selected from the group consisting of Mg²⁺, Fe²⁺, Ni²⁺, Mn²⁺, Al³⁺, Fe³⁺, and Li⁺; and
Z represents one or more ions selected from the group consisting of Al³⁺, Si⁴⁺, Ge⁴⁺, Fe³⁺, and B³⁺.

Specific examples thereof include the following:

KMg₃(AlSi₃O₁₀)F₂ Potassium phlogopite,

KMg_{2 1/2}(Si₄O₁₀)F₂ Potassium tetrasilicic mica,

KMg₂Li(Si₄O₁₀)F₂ Potassium taeniolite,

K_{2/3}Mg_{2 1/3}Li_{2/3}(Si₄O₁₀)F₂,

NaMg₃(AlSi₃O₁₀)F₂ Sodium phlogopite,

NaMg₂Li(Si₄O₁₀)F₂ Sodium taeniolite,

NaMg_{2 ½}(Si₄O₁₀)F₂ Sodium tetrasilicic mica, and

Na_{1/3}Mg_{2 2/3}Li_{1/3}(Si₄O₁₀)F₂ Sodium hectorite.

The general production method of synthetic mica powder is as follows. Layered crystals with several mm to several cm, obtained by a melting synthesis method, are coarsely crushed with a dry crusher such as a jaw crusher or hammer crusher, and then further crushed with a fine grinding machine. For example, in the case of synthetic fluorphlogopite, about 40 parts of silicic anhydride, about 30 parts of magnesium oxide, about 13 parts of aluminum oxide, and about 17 parts of potassium fluorosilicate are mixed, melted at 1,400 to 1,500 °C, and crystallized out at 1,300 to 1,400 °C to obtain synthetic fluorphlogopite. Lumps of the obtained synthetic fluorphlogopite are crushed and, if necessary, classified to obtain synthetic mica powder.

In the cosmetic application, the synthetic mica powder with the fluoride ion-elution amount of 20 ppm or less by the elution test in hot water at 100 °C for 1 hour is used. As the method to decrease the fluoride ion-elution amount to 20 ppm or less, a publicly known technology can be used. One example of a preferable method is a high-temperature heat treatment method at 600 to 1350 °C, which is shown in the above Patent Literature 1.
The pretreatment process of the present invention and the below-described coating treatment process do not affect the fluoride ion-elution amount, and the fluoride ion-elution amount of the raw material synthetic mica powder can be maintained.
The particle size of the synthetic mica powder of the present invention is not limited in particular and it can be suitably selected. Generally, the synthetic mica powder with an average particle size of 5 to 50 µm and an aspect ratio of 2 to 300 is preferably used.

### <Coating Process>

In the coating process, an organic surface treatment agent is attached on the surface of the pretreated synthetic mica powder as described above, and then baked by heating to obtain the organic-coated synthetic mica powder.

The method of attaching an organic surface treatment agent on the surface of the synthetic mica powder is not limited in particular, and a publicly known method can be used. Examples thereof include the method in which an organic surface treatment agent is mixed or sprayed to the synthetic mica powder and the method of immersion. Alternatively, it is possible that an organic surface treatment agent dissolved in a volatile solvent is subjected to mixing, spraying or immersion with the synthetic mica powder, and then the volatile solvent is volatilized. In addition, in order to achieve uniform attaching, the agitation can be carried out by suitable mechanical mixing means (for example, a kneader or ball mill). Attaching can be normally carried out at room temperature.

The organic surface treatment agent that is used in the present invention can be any agent publicly known as a water-repellent agent. Examples thereof include an organosilicone, a silane coupling agent, a titanate coupling agent, an alminate coupling agent, and a perfluoroalkylalkoxysilane. Also, the organic surface treatment agent may be a mixture of two or more compounds.

Specific examples of the organosilicone include dimethicone, methicone, (dimethicone/methicone)copolymer, triethoxysilylethylpolydimethylsiloxyethyldimethicone, triethoxysilylethylpolydimethylsiloxyethylhexyldimethicone, (acrylates/tridecyl acrylate/triethoxysilylpropyl methacrylate/dimethicone methacrylate)copolymer, modified silicones such as PEG-11 methyl ether dimethicone, polyglyceryl-3-disiloxane dimethicone, (stearoxymethicone/dimethicone)copolymer and PEGIPPG-1013 oleyl ether dimethicone, trimethylsiloxysilicate, methylphenylsilicone, dimethicone crosspolymer, (dimethicone/vinyldimethicone)crosspolymer, acrylsilicones such as (alkyl acrylate/dimethicone)copolymer, and amino modified silicones such as (aminoethylaminopropylmethicone/dimethicone)copolymer.

Specific examples of the silane coupling agent include methyltrimethoxysilane, methyltriethoxysilane, ethyltrimethoxysilane, ethyltriethoxysilane, dimethyldimethoxysilane, tetraethoxysilane, dimethyldiethoxysilane, isobutyltrimethaxysilane, n-decyltrimethaxysilane, octyltrimethoxysilane, octadecyltriethoxysilane, octyltriethoxysilane, and triethoxycaprylylsilane:

Specific examples of the titanate coupling agent include isopropoxytitanium tristearate, triisopropoxytitanium isostearate, isopropoxytitanium tripalmitate, and isopropoxytitanium trimyristate.

Specific examples of the aluminate coupling agent include acetoalkoxyaluminum diisopropylate.

Specific examples of the perfluoroalkylalkoxysilane include trifluoropropyltrimethoxysilane, trifluoropropyltriethoxysilane, tridecafluorooctyltrimethoxysilane, and tridecafluorooctyltriethoxysilane.

When the coating treatment of the conventional synthetic mica powder with such an organic surface treatment agent is carried out, the satisfactory water-repellent property cannot be obtained because of low surface reactivity of the synthetic mica. Even when the baking for a longer time and/or at a higher temperature is carried out to promote the reaction of the organic surface treatment agent, only the feeling in use is lowered due to the decrease of the amount of organochains in the organic coating layer, and it has been difficult to obtain a satisfactory water-repellent property.

In the case of the synthetic mica powder pretreated by the specific method of the present invention, the surface reactivity is enhanced. Therefore, even such an organic surface treatment agent reacts at a relatively low temperature and in a short time, and a coating layer in which the residual organochain ratio is maintained relatively high can be firmly formed. Thus, an organic-coated synthetic mica powder having a soft feeling in use and a water-repellent property can be obtained.

The baking temperature can normally be set in the range of 100 °C to 260 °C. When a silane coupling agent, a titanate coupling agent, an aluminate coupling agent, or a perfluoroalkylalkoxysilane is used as the organic surface treatment agent, the baking temperature is preferably 100°C to 170 °C. If the baking temperature is too low, the organic surface treatment agent does not react sufficiently and the water-repellent property may not be obtained. On the other hand, if the baking temperature is too high, the crosslinking polymerization reaction of the organic surface treatment agent to each other takes place excessively, and the residual carbon ratio decreases. As a result, the feeling in use becomes rough and stiff, and the water-repellent property is lowered.

When the organic surface treatment agent is an organosilicone, the baking temperature is preferably 170 to 260 °C. If the baking temperature is too low, the organic surface treatment agent does not react sufficiently and the water-repellent property may not be obtained. In the case of an organosilicone having Si-H groups, if the baking temperature is too low, the amount of Si-H groups cannot sufficiently be decreased and the value of less than 0.1 mL/g can not be achieved. On the other hand, if the baking temperature is too high, the crosslinking polymerization reaction of the organosilicone itself takes place excessively, and the residual carbon ratio decreases. As a result, the feeling in use becomes rough and stiff, and the water-repellent property is lowered.

As the organosilicone having Si-H groups, publicly known organosilicones can be used. It can be a mixture of two or more silicone compounds. Preferable examples thereof include the compounds wherein the mole ratio of Si(R)₂O unit : SiH(R)O unit in the organosilicone is in the range of 1:0.05 to 1:0.30. R represents a monovalent hydrocarbon group normally adopted in organohydrogenpolysiloxanes. Examples thereof include hydrocarbon groups having 1 to 6 carbon atoms. The typical examples thereof include a methyl group and a phenyl group. In the present invention, a methyl group is particularly preferable.

Examples of such organosilicone coating agents include methylhydrogenpolysiloxanes represented by the following formula (1): 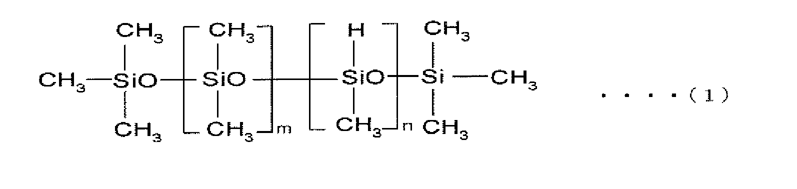 In the formula, m + n = 20 to 30, and the m : n ratio is 1:0.05 to 1:0.30.

Alternatively, as the organosilicone, a mixture of a methylhydrogenpolysiloxane, having one or more Si-H groups and with the polymerization degree of 30 or less, and a dimethylpolysiloxane without Si-H groups, wherein the mole ratio of Si(CH₃)₂O unit : SiH(CH₃)O unit, as a whole mixture, is in the range of 1:0.05 to 1:0.30, can be used.

Such organosilicones have a high content of organochains; however, the reactivity is relatively low because of a low content of Si-H groups. Therefore, when the coating treatment of the conventional synthetic mica powder with such an organosilicone is carried out, Si-H groups tend to remain. Thus, in order to decrease the hydrogen evolution amount, the baking for a longer time and/or at a higher temperature is necessary. As a result, the amount of organochains in the coating layer decreases, and it has been difficult to obtain a good feeling in use and a satisfactory water-repellent property.
In the case of the synthetic mica powder pretreated by the method of the present invention, the surface reactivity is enhanced. Therefore, even such organosilicones can be reacted at a relatively low temperature and in a short time to lose the Si-H groups and the organic coating layer in which the residual organochain ratio is maintained relatively high can be firmly formed on the powder surface. As a result, the organic-coated synthetic mica powder having a soft feeling in use and a water-repellent property can be obtained.

The amount of used organic surface treatment agent is not limited in particular. However, it is preferable to use the amount that can coat the entire surface of the synthetic mica. Normally, 0.5 to 10 mass % of the organic surface treatment agent is used with respect to 100 mass % of the synthetic mica powder as the raw material, and preferably 1 to 5 mass % of the organic surface treatment agent is used. If the amount used is too small, the water-repellent property may be unsatisfactory. On the other hand, even when an excess amount is used, an increase in the effect cannot be expected and, on the contrary, the texture of the obtained organic-coated synthetic mica powder may be undermined due to the aggregation of synthetic mica powder particles caused by attaching excess organic surface treatment agent on the synthetic mica powder. In the case of an organosilicone having Si-H groups, if an excess amount is used, it may be difficult to achieve the value of 0.1 mL/g or less.

In the thus obtained organic-coated synthetic mica powder of the present invention, the organic coating layer is firmly bound to the powder surface and does not peel off. In addition, the residual organochain ratio in the organic coating layer is relatively high. Therefore, it has a soft feeling in use without roughness and stiffness, and excellent water-repellent property. In addition, even when an organosilicone having Si-H groups is used, the hydrogen evolution with time does not take place because Si-H groups hardly remain.
Furthermore, if the synthetic mica powder with the fluoride ion-elution amount of 20 ppm or less is used as the starting raw material, the fluoride ion-elution amount can be maintained 20 ppm or less because there is no increase in the fluoride ion-elution amount due to the pretreatment and coating treatment.
Therefore, the organosilicone-coated synthetic mica powder of the present invention can be suitably used for cosmetic application, and in particular, useful in makeup cosmetics such as foundations and face powder.

### EXAMPLES

Hereinafter, the present invention will be further explained with reference to specific examples. However, the present invention is not limited by these examples. The test methods used in the present invention are as follows.

### Test Methods

### (Surface Activity)

Into a Pyrex^{®} glass tube with an inner diameter of 4 mm, 20 mg of powder was fixed with quartz wool. 0.3 µL of t-butanol was passed through the tube at 250°C, and the decomposition rate thereof was measured. For the analysis, a gas chromatograph GC-14B manufactured by Shimadzu Corporation was used. The column was PEG-20M (3 mm x 2 m), the column temperature was 80 °C, the flow rate was 30 mL/min, and nitrogen was used as the carrier gas. The evaluation of surface activity was expressed with the t-butanol decomposition rate. The larger the value of the decomposition rate, the higher the surface activity; while the smaller the value, the lower the surface activity.

### (Hydrogen Evolution Amount)

At ordinary temperature and under atmospheric pressure, 10 g of organosilicone-coated synthetic mica powder was placed in a container, and 5 mL of benzene was added. Subsequently, hydrogen gas was generated by the dropwise addition of 26 mass % potassium hydroxide/ethanol solution in the closed system. Then the hydrogen gas-evolution amount per unit mass (mL/g) was calculated for the coated powder.

### (Residual Carbon Ratio)

Into a nickel capsule, 0.1 g of synthetic mica powder coated with an organic surface treatment agent was enclosed. It was placed in a ceramic crucible with tungsten combustion improver. With a high-frequency induction furnace type carbon and sulfur analyzer EMIA-520, manufactured by Horiba, Ltd., the carbon amount in the powder was measured. The residual carbon ratio (mass %) with respect to the carbon amount before baking, which was calculated from the amount of used organic surface treatment agent, was calculated.

### (Fluoride Ion-Elution Amount)

The test was carried out according to the measurement of fluoride elution amount in the Japan Quasi-drug Raw Material Specification 2006. 5 g of test powder was placed into a flask, and 100 mL of water was added thereto. The mixture was refluxed by heating for 1 hour and then filtrated. The lanthanum-alizarin complexone reagent, acetone and water was added to the filtrate, and then the absorbance was measured. The fluoride ion-elution amount (ppm) with respect to the mass of the test powder was calculated by comparing with the blank.

### (Feeling in Use)

The feeling in use on the skin was evaluated based on the following criteria.
○: good (excellent softness without roughness and stiffness)
Δ: slightly bad (inferior softness with some roughness and stiffness)
×: bad (no softness with roughness and stiffness)

### (Water-Repellent Property)

10 mL of ion-exchanged water was placed into a sealable test tube with a lid, and 0.1 g of sample powder was added thereto. The mixture was manually shaken, allowed to stand for 1 day, and then the turbidity was evaluated as the water-repellent property. The evaluation criteria are as follows.
○: good (the powder is aggregated in the gas-liquid interface, and the water phase is transparent.)
Δ: slightly bad (the water phase is slightly turbid, and a portion of the powder is precipitated sometimes.)
x: bad (the water phase is turbid, and a portion of the powder is suspended in the water phase but the most powder is precipitated, or the entire powder is precipitated.)

The pretreatment and the coating treatment in the respective tests were carried out according to the following methods.

### (1) Pretreatment

100 g of synthetic fluorphlogopite powder (PDM-10S manufactured by TOPY KOGYO KABUSHIKIKAISYA, average particle size: 12 µm, fluoride elution amount: 14 ppm) and 900 g of water were mixed and stirred to prepare a slurry. A pretreatment agent was added to the slurry and stirred at room temperature for 10 minutes. Subsequently, the solid-liquid separation was carried out by filtration to remove the liquid phase. The obtained solid phase was dried at 150 °C for 3 hours to volatilize water and NH₃.

### (2) Coating Treatment

3 g of organic surface treatment agent dissolved in 3 g of solvent was added and mixed to 97 g of dry synthetic mica powder to attach uniformly on the powder. The obtained powder was baked at 230 °C to give organic-coated synthetic mica powder.

### Test Example 1 Case without Pretreatment

Table 1 shows the residual carbon ratio, hydrogen evolution amount, feeling in use, and the water-repellent property of the organic-coated synthetic mica powder obtained by the coating treatment without pretreatment.
As the organic surface treatment agent, a mixture of 0.55 g of methylhydrogenpolysiloxane (KF-9901 manufactured by Shin-Etsu Chemical Co., Ltd., polymerization degree: 20 to 30 (specification value)) and 2.45 g of dimethylpolysiloxane (KF-96A-30cs manufactured by Shin-Etsu Chemical Co., Ltd., polymerization degree: about 20), wherein the mole ratio of Si(R)₂O unit : SiH(R)O unit was 1:0.11, was used. As the solvent, isopropyl alcohol was used.

**Table 1**

| Baking time hr | residual carbon ratio % | hydrogen-evolution amount ml/g | Feeling in use | Water - repellent property |
|---|---|---|---|---|
| 3 | 61 | 0.31 | ○ | ○ |
| 6 | 52 | 0.16 | ○ | ○ |
| 10 | 39 | <0.1 | Δ | Δ |

As shown in Table 1, when a conventional synthetic mica powder was coated with an organosilicone having Si-H groups, the powder with a good feeling in use and a good water-repellent property could be obtained; however, the hydrogen evolution amount was large. When the hydrogen evolution amount was suppressed to less than 0.1 mL/g, the amount of organochains in the coating layer decreased because of the crosslinking polymerization reaction of the organic surface treatment agent to each other, which resulted in lowering of the feeling in use and the water-repellent property.
Thus, the pretreatment of synthetic mica powder was investigated.

### Test Example 2 Effect of Pretreatment Agent

Table 2 shows the evaluation results for the t-butanol decomposition rate and the fluoride ion-elution amount of the synthetic mica powder obtained by the treatment of various pretreatment agents. The used pretreatment agents and the used amounts are as follows.
25% Aqueous ammonia: 0.5, 1, 10, 30 mass % in the amount of nitrogen with respect to the synthetic mica.
Ammonium hydrogencarbonate: 10 mass % in the amount of nitrogen with respect to the synthetic mica.
Ammonium carbonate: 10 mass % in the amount of nitrogen with respect to the synthetic mica.
Potassium hydroxide: 10 mass % with respect to the synthetic mica.
Citric acid: 10 mass % with respect to the synthetic mica.

**Table 2**

| Test Example | 2-1 | 2-2 | 2-3 | 2-4 | 2-5 | 2-6 | 2-7 | 2-8 | 2-9 | 2-10 |
|---|---|---|---|---|---|---|---|---|---|---|
| Synthetic mica PDM-10S(g) | 100 | 100 | 100 | 100 | - | 100 | 100 | 100 | 100 | 100 |
| Synthetic mica PDM-9WA(g)* | - | - | - | - | 100 | - | - | - | - | - |
| Water(g) | 900 | 900 | 900 | 900 | 900 | 900 | 900 | 900 | 900 | 900 |
| 25% Aqueous ammonia (g) | 2.4 | 4.9 | 48.6 | 145.9 | 48.6 | - | - | - | - | - |
| (in the amount of N) | 0.5 | 1 | 10 | 30 | 10 | - | - | - | - | - |
| Ammonium | | | | | | | | | | |
| hydrogencarbonate (g) (in the amount of N) | - | - | - | - | - | 56.4 | - | - | - | - |
| | - | - | - | - | - | 10 | - | - | - | - |
| Ammonium carbonate (g) (in the amount of N) | - | - | - | - | - | - | 34.3 | - | - | - |
| | - | - | - | - | - | - | 10 | - | - | - |
| KOH(g) | - | - | - | - | - | - | - | 10.0 | - | - |
| Citric acid (g) | - | - | - | - | - | - | - | - | 10.0 | - |
| t-Butanol decomposition rate (%) | 8 | 40 | 65 | 62 | 59 | 64 | 63 | 64 | 4 | 3 |
| F-elution amount (ppm) | 12 | 12 | 13 | 13 | 15 | 10 | 12 | 15 | 60 | 12 |

| | | | | | | | | | | |
|---|---|---|---|---|---|---|---|---|---|---|
| * P D M - 9 W A : manufactured by TOPY KOGYO KABUSHIKIKAISYA, average particle size: 12 µm, F-elution amount: 15ppm | | | | | | | | | | |

As shown in Test Example 2-10, when no pretreatment agent was used, the surface activity was hardly observed.
On the other hand, as shown in Test Examples 2-1 to 2-7, the pretreatment with ammonium ions improved the surface activity regardless of the kinds of pretreatment agents. Especially when the pretreatment was carried out with ammonium ions in the amount of 1 to 30 mass % nitrogen with respect to the amount of synthetic mica, the surface activity improved significantly. An increase in the fluoride ion-elution amount due to pretreatment was not observed.
As shown in Test Example 2-8, when potassium hydroxide was used as the pretreatment agent, the improvement effect of surface activity was also observed, similar to the use of ammonium ions, without an increase in the fluoride ion-elution amount.
On the other hand, as shown in Test Example 2-9, when citric acid was used as the pretreatment agent, the improvement effect of surface activity was not observed, and the fluoride ion-elution amount increased drastically.

Next, using the dry synthetic mica powder obtained by the pretreatment as the powder to be coated, the coating treatment was carried out in the same way as the above-described Test Example 1, to obtain the organic-coated synthetic mica powder. The results after the baking was carried out at 230 °C for 1 hour are shown in Table 3.

**Table 3**

| Test Example No. | 2-3a | 2-5a | 2-6a | 2-7a | 2-8a | 2-10a |
|---|---|---|---|---|---|---|
| Synthetic mica used in coating treatment | Test Exemple 2-3 | Test Example 2-5 | Test Example 2-6 | Test Example 2-7 | Test Example 2-8 | Test Example 2-10 |
| (Pretreatment agent) | 25% Aqueous ammonia | 25% Aqueous ammonia | Ammonium hydrogencarbonate | Ammonium carbonate | KOH | - |
| residual carbon ratio (%) | 51 | 52 | 53 | 48 | 9 | 72 |
| H₂ evolution amount (ml/g) | <0.1 | <0.1 | <0.1 | <0.1 | <0.1 | 0.4 |
| Feeling in use | ○ | ○ | ○ | ○ | × | ○ |
| Water-reppellent property | ○ | ○ | ○ | ○ | × | ○ |

As shown in Table 3, when no pretreatment agent was used, the feeling in use and the water-repellent property after the baking treatment at 230 °C for 1 hour were good; however, the hydrogen evolution amount was large (Test Example 2-10a).
On the other hand, when the synthetic mica powder pretreated with ammonia, ammonium hydrogencarbonate, or ammonium carbonate was used, the organic-coated synthetic mica powder exerting good feeling in use and a good water-repellent property with an extremely low hydrogen evolution amount of less than 0.1 mL/g could be obtained by the baking treatment at 230 °C for 1 hour (Test Example 2-3a and Test Examples 2-5a to 2-7a).

On the other hand, when the synthetic mica powder pretreated with KOH was used, an organic-coated synthetic mica powder with a hydrogen evolution amount of less than 0.1 mL/g could be obtained: however, the texture was stiff and rough, and no water-repellent property was observed (Test Example 2-8a).
Thus, it has been clarified that an organic-coated synthetic mica powder having very good feeling in use and a very good water-repellent property without hydrogen evolution can easily be obtained by the pretreatment of synthetic mica powder with ammonium ions to activate the surface of the powder.

### Test Example 3 Hydrogen Evolution Amount and Residual Carbon Ratio

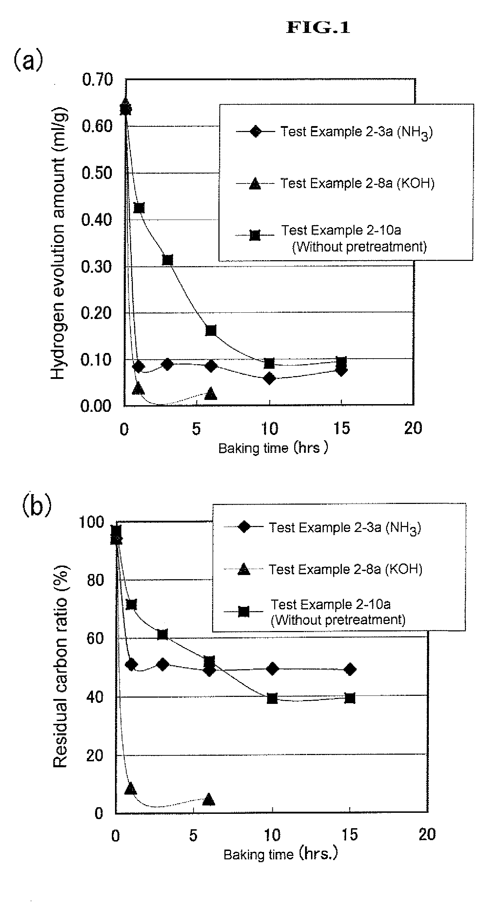

Fig. 1 shows the results of (a) the hydrogen evolution amount and (b) the residual carbon ratio for the obtained organic-coated synthetic mica powder when the baking time was varied in Test Example 2.

As also seen from Fig. 1, in the case of no pretreatment (Test Example 2-10a), the hydrogen evolution amount gradually decreased if the baking time was lengthened. By baking for 10 hours, the hydrogen evolution amount could be decreased to less than 0.1 mL/g. Therefore, even without pretreatment, the hydrogen evolution amount could sufficiently be decreased if the baking treatment was carried out for a longer time. However, the residual carbon ratio gradually decreased with an increase in the baking time and became lower than 40% by baking for 10 hours. As shown in the above Table 1, when the residual carbon ratio became lower than 40%, the roughness and stiffness were felt, the softness in texture became inferior, and the water-repellent property was lowered.

On the other hand, when the pretreatment was carried out with ammonia (Test Example 2-3a), the hydrogen evolution amount rapidly decreased to less than 0.1 mL/g by baking for 1 hour. On this occasion, the residual carbon ratio decreased to about 50% of the content before baking: however, it did not decrease any more and was maintained constant even when the baking time became longer. Thus, the good feeling in use and the good water-repellent property could be maintained. When the pretreatment was carried out with ammonium hydrogencarbonate or ammonium carbonate, the same trend was observed as the case of the pretreatment with ammonia.

On the other hand, when the pretreatment was carried out with the use of KOH (Test Example 2-8a), the hydrogen evolution amount could be decreased to less than 0.1 mL/g, like the treatment with ammonia, by baking for 1 hour. At the same time, however, the residual carbon amount significantly decreased to about 10% of the content before baking, which resulted in significant lowering of the feeling in use and the water-repellent property.

The cause of such a phenomenon is not clear; however, the following presumption is possible. That is, if no pretreatment is carried out, the Si-H groups remain because of low reactivity of the surface of the synthetic mica powder. In order to decrease the amount of Si-H groups, the excess baking is necessary. As a result, the excessive crosslinking polymerization of the organosilicone to each other takes place, and the residual carbon ratio decreases.

On the other hand, when the pretreatment with ammonium ions is carried out, the surface of the synthetic mica powder is activated. Therefore, Si-H groups easily react on the powder surface to be consumed. As a result, the excessive crosslinking polymerization of the organosilicone to each other is suppressed. Thus, the decrease of Si-H groups as well as the remain of carbon amount can be achieved.

On the other hand, when the pretreatment with KOH is carried out, the strong alkali KOH remains on the powder surface. Thus, KOH functions as a catalyst to significantly promote the crosslinking polymerization of the organosilicone to each other in the baking treatment. As a result, the residual carbon ratio significantly decreases to be compositionally- close to silica.

### Test Example 4 Baking Temperature

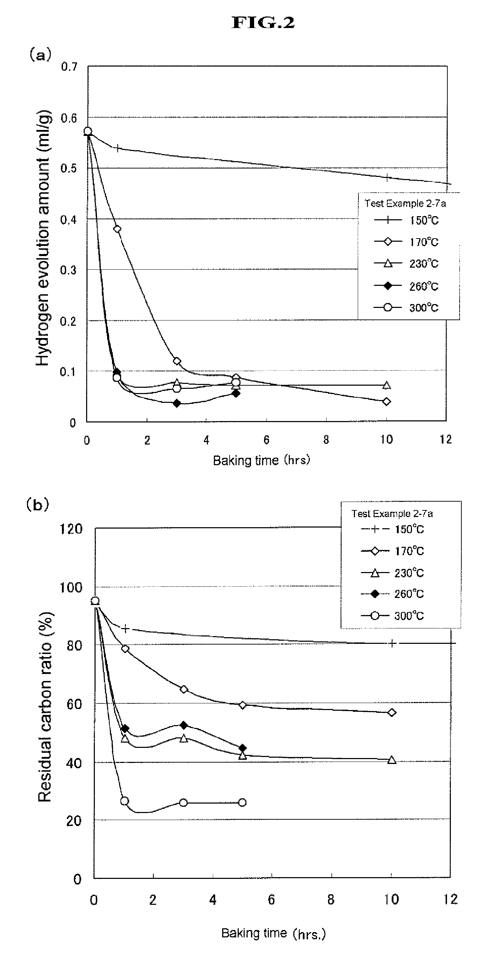

Fig. 2 shows (a) the hydrogen evolution amount and (b) the residual carbon ratio when the baking temperature and time were varied in Test Example 2-7a (pretreatment agent: ammonium carbonate).
As seen from Fig. 2, when the baking temperature was 300 °C, the hydrogen evolution amount could be decreased to less than 0.1 mL/g by baking for 1 hour. At the same time, however, the residual carbon ratio significantly decreased to about 25% of the content before baking, and the feeling in use and the water-repellent property became unsatisfactory.
On the other hand, when the baking temperature was 150 °C, the hydrogen evolution amount could not be decreased to less than 0.1 mL/g even by baking for 15 hours or longer.

In contrast, when the baking temperature was 170 to 260 °C, the hydrogen evolution amount could be decreased to less than 0.1 mL/g within a relatively short time (for example, within 10 hours). In addition, the residual carbon ratio could be maintained at 40% or higher of the content before baking, and the good feeling in use and the good water-repellent property were obtained.
Therefore, in the case of organosilicone, in order to obtain an organic-coated synthetic mica powder with a hydrogen evolution amount of less than 0.1 mL/g and having a good feeling in use and a good water-repellent property, the baking temperature is preferably in the range of 170 to 260 °C.

As shown in the below Table 4, when the residual carbon ratio decreased, the feeling in use also decreased. When the residual carbon ratio became lower than 40%, the roughness and stiffness were felt, and the softness in texture became inferior. In addition, when the residual carbon ratio decreased, the water-repellent property decreased.
Therefore, the residual carbon ratio is preferably 40% or higher, and more preferably 45% or higher.
On the other hand, as also seen from Figs. 1 to 2, if we try to maintain the residual carbon at an excessively high percentage, the hydrogen evolution amount cannot be lowered to less than 0.1 mL/g; thus it is preferable to allow the residual carbon ratio to be 60% or lower.

**Table 4**

| Test Example No. | Baking temperature °C | Baking time hr | Residual carbon ratio % | Hydrogen evolution amount ml/g | Feeling in use | Water-repellent property |
|---|---|---|---|---|---|---|
| 2-7a₁ | 150 | 40 | 69 | 0.36 | ○ | ○ |
| 2-7a₂ | 170 | 5 | 59 | <0.1 | ○ | ○ |
| 2-7a₃ | 230 | 1 | 48 | <0.1 | ○ | ○ |
| 2-7a₄ | 260 | 1 | 51 | <0.1 | ○ | ○ |
| 2-7a₅ | 300 | 1 | 26 | <0.1 | Δ | Δ |

### Test Example 5 Organic Surface Treatment Agent

In Test Example 2-3a, instead of the mixture of silicones, a single methylhydrogenpolysiloxane, with Si(R)₂O unit: SiH(R)O unit = 1:0.11 (mole ratio) and a polymerization degree of 25, was used to obtain an organic-coated synthetic mica powder (Test Example 2-3b).
As a result, as shown in Table 5, no difference was observed compared with the use of the mixture of methylhydrogenpolysiloxane-dimethylpolysiloxane (Test Example 2-3a), in all the aspects, namely, the residual carbon ratio, hydrogen evolution amount, feeling in use, and the water-repellent property.
Thus, when an organosilicone having Si-H groups is used as the organic surface treatment agent, either a mixture of methylhydrogenpolysiloxane and dimethylpolysiloxane or a single methylhydrogenpolysiloxane can equally be used so far as the mole ratio between Si(R)₂O unit and SiH(R)O unit is the same.

**Table 5**

| Organic surface treatment agent | Baking time hr | Residual carbon ratio % | Hydrogen evolution amount ml/g | Feeling in use | Water-repellent property |
|---|---|---|---|---|---|
| Mixture of methylhydrogenpolysiloxane + dimethylpolysiloxane | 1 | 51 | <0.1 | ○ | ○ |
| | 5 | 51 | < 0.1 | ○ | ○ |
| | 10 | 53 | <0.1 | ○ | ○ |
| Methylhydrogenpolysiloxane only | 1 | 53 | <0.1 | ○ | ○ |
| | 5 | 54 | <0.1 | ○ | ○ |
| | 10 | 52 | <0.1 | ○ | ○ |

The Table 6 below shows the results when the baking treatment was carried out at 230 °C for 1 hour in Test Example 2-3a (pretreatment agent: ammonia) or Test Example 2-10a (without a pretreatment agent), using an organosilicone having no Si-H groups (however, in Test Examples 2-3f, 2-3g, 2-10f and 2-10g, in which an organic surface treatment agent other than organosilicones was used, the treatment was carried out at 120°C for 1 hour).

As shown in Table 6, when the conventional synthetic mica powder with low surface activity was used, an organic-coated synthetic mica powder with a high water-repellent property could not be obtained by the coating treatment with any of the organic surface treatment agents (Test Examples 2-10c to 2-10g). As also seen from Table 7, even when the baking temperature was increased or the baking time was lengthened, only the amount of organochains decreased, leading to the decrease in the feeling in use, and the water-repellent property was not improved. This is considered because only the crosslinking polymerization of the organic surface treatment agent is promoted, and the bonding of the coating layer to the powder surface is not promoted.

On the other hand, in the case of highly surface active synthetic mica powder obtained by the pretreatment method of the present invention, an organic-coated synthetic mica powder, with the residual carbon ratio of 40 to 60%, which has a good feeling in use and excellent water-repellent property, could easily be obtained (Test Examples 2-3c to 2-3g).
In addition, it was possible to bake at a lower temperature in the case of the silane coupling agents such as those in Test Examples 2-3f and 2-3g.

**Table 6**

| Test Example No. | 2-3c | 2-3d | 2-3e | 2-3f | 2-3g |
|---|---|---|---|---|---|
| Pretreatment agent | 25% Aqueous ammonia | 25% Aqueous ammonia | 25% Aqueous ammonia | 25% Aqueous ammonia | 25% Aqueous ammonia |
| Organic surface treatment agent * | Dimethicone | Triethoxysilylethyl Dimethicone polydimethylsiloxyethyl dimethicone | crosspolymer | Octyltriethoxysilane | Tridecafluorooctyl-triethoxysilane |
| Solvent * | Cyclohexane | IPA | Cyclohexane | IPA | IPA |
| Residual carbon ratio (%) | 51 | 52 | 55 | 60 | 50 |
| Feeling in use | ○ | ○ | ○ | ○ | ○ |
| Water-repellent property | ○ | ○ | ○ | ○ | ○ |
| | | | | | |

| Test Example No. | 2-10c | 2-10d | 2-10e | 2-10f | 2-10g |
|---|---|---|---|---|---|
| Pretreatment agent | - | - | - | - | - |
| Organic surface treatment agent * | Dimethicone | Triethoxysilyethyl polydimethylsiloxyethyl dimethicone | Dimethicone crosspolymer | Octyltriethoxysilane | Tridecafluorooctyl-Tridecafluorooctyl-triethoxysilane |
| Solvent * | Cyclohexane | IPA | Cyclohexane | IPA | IPA |
| Residual carbon ratio (%) | 70 | 65 | 72 | 18 | 23 |
| Feeling in use | ○ | ○ | ○ | × | Δ |
| Water-repellent property | × | × | × | × | × |

| | | | | | |
|---|---|---|---|---|---|
| *Organic surface treatment agent. Solvent Dimethicone: manufactured by Shin-Etsu Chemical Co., Ltd., KF-96-1, 000CS Tiethoxysilylethyl polydimethylsiloxyethyl dimethicone: manufactured by Shin-Etsu Chemical Co., Ltd., K-9908 Dimethicone crosspolymer: manufactured by Dow Corning Toray Co., Ltd., 9045 Silicone Elastomer Blend Octyltriethoxysilane: manufactured by Shin-Etsu Chemical Co., Ltd., AES-3083 Tridecafluorooctyltriethoxysilane: manufactured by Evonik Degussa Japan Co., Ltd., DYNASYLAN F8261 IPA : isopropyl alcohol | | | | | |

**Table 7**

| Pretreatment :None | | | |
|---|---|---|---|
| Organic surface treatment agent : dimethicone (solvent :cyclohexane) | | | |
| Baking Condition | Residual carbon ratio (%) | Feeling in use | Water-repellent property |
| 230°C × 1 hour | 70 | ○ | x |
| 230°C × 5 hours | 48 | ○ | × |
| 230°C × 10 hours | 35 | Δ | × |
| 230°C × 20 hours | 20 | × | × |
| 250°C × 1 hour | 50 | ○ | × |
| 300°C × 1 hour | 20 | × | × |

### Test Example 6 Removal of Interlayer Cations in Pretreatment

In order to investigate what effect is exerted to the surface of synthetic mica by the pretreatment, the elements dissolved in the liquid phase, which was removed in the pretreatment of the above-described Test Example 2, were analyzed. The concentration of dissolved element was determined by the gravimetric method for Si, or by ICP emission spectrometry for other elements. The results are shown in Table 8.

**Table 8**

| Dissolved element | NH₃ treatment | Ammonium carbonate treatment | Citric acid treatment | Pure water treatment |
|---|---|---|---|---|
| K | 0.43 | 0.49 | 0.49 | 0.25 |
| Mg | 0.14 | 0.05 | 1.85 | 0.11 |
| Al | 0.08 | 0.00 | 1.37 | 0.04 |
| Si | 1.53 | 0.57 | 1.32 | 0.30 |

| | | | | |
|---|---|---|---|---|
| (Unit mmol/L) | | | | |

As shown in Table 8, in the pretreatment with ammonium ions (NH₃ or ammonium carbonate), compared with the treatment with pure water, there was little difference in the elution amount of octahedral elements (Mg, Al), which has been present inside the synthetic mica. However, the elution amounts were large in tetrahedral element (Si), which has been present on the surface of the synthetic mica, as well as interlayer cations (K), which has been adsorbed on the tetrahedral layer surface.
On the other hand, in the case of citric acid treatment, the elution amounts were large in both interior elements (Mg, Al) and surface elements (K, Si).
Thus, it is considered that: the action of acid treatment is not only on the synthetic mica surface (i. e., (001) face), but also on the end face, and therefore, fluoride ions are easily eluted from the end face; on the other hand, the action of ammonium ion treatment is only on the surface of the synthetic mica, and therefore, hardly affect the elution of fluoride ions.

Furthermore, the percentage of K atoms eluted into the pretreatment waste liquid (about 0.9 L) with respect to the total number of K atoms, which has been present on the surface of the synthetic mica, was calculated. As shown in Table 9, 30% or higher of K atoms on the surface of the synthetic mica were removed by the ammonium ion treatment. It is considered that K ions on the powder surface were replaced with ammonium ions, and the liberated K ions were removed to the outside of the system by the liquid phase removal.
The interlayer cations adsorbed on the surface of the synthetic mica powder become a hindrance when an organic surface treatment agent approaches the tetrahedral layer. Therefore, it is speculated that the removal of interlayer cations by ammonium ion treatment contributes, in addition to the reactive site formation, to the improvement of the reactivity between the surface of the synthetic mica powder and the organic surface treatment agent.
Therefore, it is preferable to remove 20% or higher of interlayer ions on the surface of the synthetic mica powder in the pretreatment, and more preferably 30% or higher.

**Table 9**

| | **NH₃** | Ammonium carbonate | Pure water |
|---|---|---|---|
| K-elution amount (%) | **33.2** | **37.1** | **18.7** |

| | | | |
|---|---|---|---|
| * Calculation method of the amount of K that has been present on the synthetic mica surface: Specific surface area of (001) face (3.47 m²/g) = BET specific surface area (measured value: 3.6 m²/g) - specific surface area of end face (theoretical value: 0.13 m²/g); Based on the Wyckoff lattice constant (a = 5.31 Å, b = 9.21 Å from Crystal Structure, Vol. 4, p. 344, 1966), the number of surface K per 100g of powder is calculated by assuming that one K atom is present per the (001) face with 5.31 Å×9.21 Å as follows, Number of surface K (number/100 g powder) = (3.47 × 100)/(5.31 × 9.21 × 10⁻²⁰) = 7.1 × 10²⁰; and The specific surface area of the end face was calculated from the average value of the particle thickness measured with an electron microscope and the particle size distribution values measured with a laser diffraction particle size distribution analyzer for the used | | | |

### Test Example 7 Drying Temperature in Pretreatment

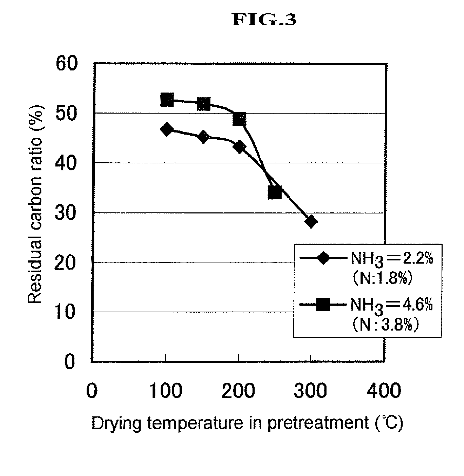

Fig. 3 shows the change in the residual carbon ratio when the drying temperature was varied in the pretreatment. In Fig. 3, the NH₃ concentration used in the pretreatment was 2.2 mass % or 4.6 mass % (1.8 mass % or 3.8 mass % in the amount ofN) with respect to the amount of synthetic mica powder. Other conditions were the same as those of the above-described Test Example 2-3a except for that the baking time was 5 hours.

As seen from Fig. 3, if the drying temperature after the dewatering from the slurry is too high, the residual carbon ratio in the obtained organic-coated synthetic mica powder decreases significantly.
The definite reason for this is not known; however, the following presumption is possible. That is, the reactive sites formed on the surface of the synthetic mica by the contact with ammonium ions decreases by high-temperature drying; as a result, the surface activity of synthetic mica powder decreases. In addition, as shown in the above-described Test Example 6, because the interlayer cations (potassium ions) on the surface of the synthetic mica powder are removed in the pretreatment with ammonium ions, the hindrance is reduced. When an organic surface treatment agent is baked on such a synthetic mica powder, the reaction of the organic surface treatment agent to each other takes place easily, and the residual carbon ratio is considered to decrease.
Thus, in the pretreatment of the present invention, it is important to volatilize NH₃ at a low temperature. It is preferable to dry at 200 °C or lower, and more preferably at 180 °C or lower.

### Test Example 8 Comparison with Natural Inorganic Powder

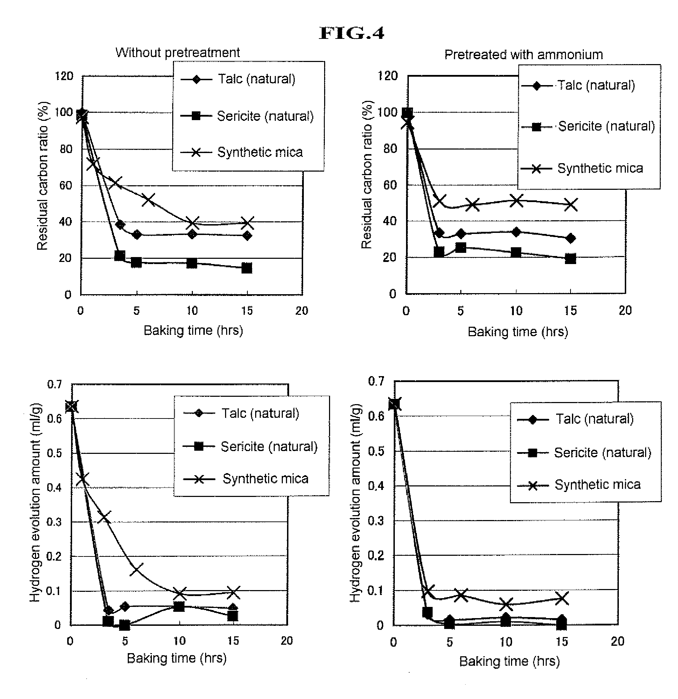

Fig. 4 shows the results when natural inorganic powder (sericite or talc) was used instead of the synthetic mica powder in Test Example 2-3a.
As shown in Fig. 4, even when the pretreatment of the present invention was carried out for the natural inorganic powder, the residual carbon ratio and the hydrogen evolution amount of the obtained organic-coated powder were hardly affected.

### Test Example 9 Effect of Solid-Liquid Separation

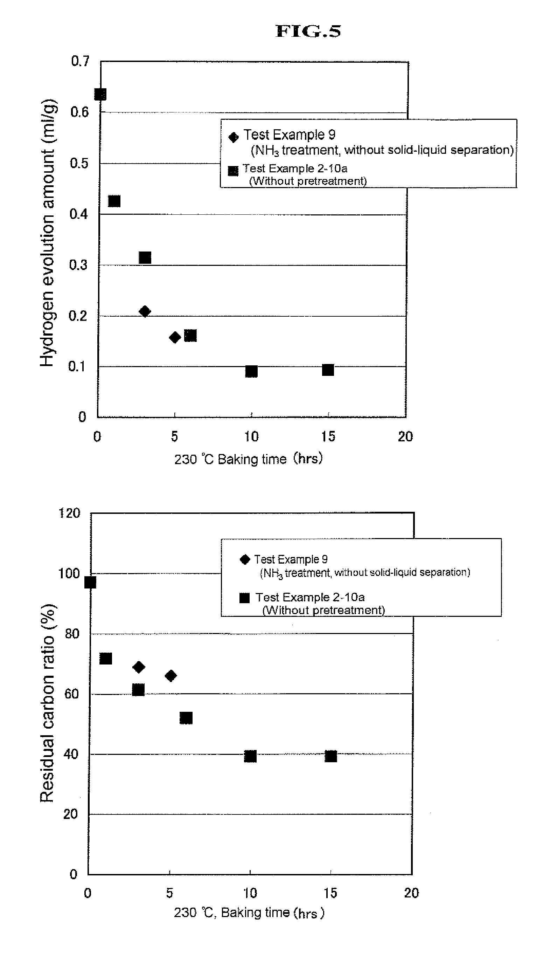

The organic-coated synthetic mica powder was obtained in the same way as Test Example 2-3a except for that the solid-liquid separation by filtration was not carried out in the pretreatment process with NH₃ and the drying at 150°C was carried out as it is.
As a result, as shown in Fig. 5, when the removal of the liquid phase by solid-liquid separation was not carried out, though the NH₃ pretreatment was carried out (Test Example 9), the residual carbon ratio and the hydrogen evolution amount displayed similar behaviors to the case without pretreatment (Test Example 2-10a); thus the effect was not observed. This reason is inferred that the removal of K ions from the surface of synthetic mica, by the removal of the liquid phase, was not sufficiently carried out.

### Blending Example 1 Powdery Foundation

The organic-coated synthetic mica powder of the above Test Example 2-3a was used as the test powder to prepare a solid powdery foundation. In addition, for comparison, the organic-coated synthetic mica powder of Test Example 2-8a or Test Example 2-7a₅, in which the residual carbon ratio is low, were used to prepare solid powdery foundations. The formulation was as follows.

<formulation>

| | |
|---|---|
| (1) Test powder | 55 parts by mass |
| (2) Titanium oxide | 7 |
| (3) Muscovite | 3 |
| (4) Talc | 20 |
| (5) Nylon powder | 2 |
| (6) Red iron oxide | 0.5 |
| (7) Yellow iron oxide | 1 |
| (8) Black iron oxide | 0.1 |
| (9) Silicone oil | 1 |
| (10) 2-Ethylhexyl palmitate | 9 |
| (11) Sorbitan sesquioleate | 1 |
| (12) Preservative | 0.3 |
| (13) Perfume | 0.1 |

### <Production method>

The components 1 to 8 were mixed with a Henschel mixer, and components 9 to 13, which had been dissolved and mixed under heating, were added and mixed thereto. After the crushing with a pulverizer, the mixture was shaped into an inner tray with a diameter of 53 mm, under a pressure of 150 kg/cm², to obtain a powdery foundation.

### (Evaluation of usability)

The sensory evaluation was carried out for the foundations prepared according to the above formulation. The softness and the spreadability when respective samples were applied on the skin were evaluated by 15 panelists according to the below five levels 1-5.
1: Bad
2: Slightly bad
3: Normal
4: Slightly good
5: Good
The results were evaluated, as described below, based on the average value of the five-level evaluations by 15 panelists.
⊚ :4.5 to 5.0
○: 3.5 to 4.4
□: 2.5 to 3.4
Δ: 1.5 to 2.4
x: 1.0 to 1.4

**Table 10**

| Test powder | Residual carbon ratio (%) | Softness | Spreadability |
|---|---|---|---|
| Test Example 2-3a | 51 | ⊚ | ○ |
| Test Example 2-8a | 9 | × | × |
| Test Example 2-7a₅ | 26 | Δ | Δ |

As also seen from Table 10, the foundation prepared with the use of the organic-coated synthetic mica powder of the present invention (Test Example 2-3a) was very soft and very spreadable and the texture was excellent, compared with the case in which the powder of the comparative examples (Test Example 2-8a, Test Example 2-7a₅) was used.

### Blending Example 2 Emulsion Foundation

The organic-coated synthetic mica powder of the above Test Example 2-3a was used as the test powder to prepare an emulsion foundation. In addition, for comparison, the organic-coated synthetic mica powder of Test Example 2-7a₁ with high hydrogen evolution amount, or that of Test Example 2-7a₅ with low residual carbon ratio, were used to emulsion foundations. The test formulation was as follows.

<Formulation>

| | | |
|---|---|---|
| (A) | Cyclopentasiloxane | 14.38 parts by mass |
| | Dimethylpolysiloxane (10 cs) | 12.00 |
| | Squalane | 4.00 |
| | Trioctanoin | 3.00 |
| | Tocopherol | Q.S. |
| | Distearyldiammoniumhectorite | 0.20 |
| (B) | Hydrophobized titanium oxide | 7.00 |
| | Hydrophobized red iron oxide | 0.20 |
| | Hydrophobized yellow iron oxide | 1.00 |
| | Hydrophobized black iron oxide | 0.20 |
| | Test powder | 15.00 |
| (C) | Ion-exchanged water | 31.70 |
| | Trisodium citrate | 2.00 |
| | 1,3-Butylene glycol | 5.00 |
| | Paraben | Q.S. |
| | Glycerin | 3.00 |

### <Production method>

The component (A) was dissolved by heating, and the powder of component (B) was added and dispersed thereto with a homomixer. The beforehand dissolved and heated component (C) was added to the dispersion, and the mixture was emulsified with a homomixer and cooed to room temperature to prepare an emulsion foundation.

### (Evaluation of usability)

For the above emulsion foundation that was stored in a tightly sealed container at 50 °C for 1 month, the sensory evaluation in the softness and the long-lasting property was carried out based on similar evaluation criteria to those of the above Blending Example 1. In addition, the change of the container was also investigated.

**Table 11**

| Test powder | Residual carbon ratio (%) | H₂ evolution amount (ml/g) | Softness | Long-lasting property | Time-dependent stability |
|---|---|---|---|---|---|
| TestExample 2-3a | 51 | <0.1 | ⊚ | ○ | No problem |
| Test Example 2-7a₅ | 26 | <0.1 | × | × | No problem |
| Test Example 2-7a₁ | 69 | 0.36 | ⊚ | ⊚ | Container expands |

As also seen from Table 11, the emulsion foundation prepared with the organic-coated synthetic mica powder of the present invention (Test Example 2-3a) was very soft and long-lasting compared with Test Example 2-7a₅. In addition, the container expansion due to hydrogen evolution was not observed unlike Test Example 2-7a₁.

## Claims

1. A method for producing organic-coated synthetic mica powder, comprising a pretreatment process and a coating process,
said pretreatment process comprising:
contacting synthetic mica powder and an ammonium ion in water;
carrying out solid-liquid separation to remove the liquid phase; and
drying the solid phase at 200 °C or lower to volatilize NH₃, and
said coating process comprising:
attaching an organic surface treatment agent on the surface of the pretreated synthetic mica powder; and
baking the organic surface treatment agent-attached synthetic mica powder to form an organic coating layer on the surface of the synthetic mica powder.

2. The method of claim 1, wherein a supply source of the ammonium ion is NH₃ or a water-soluble ammonium salt.

3. The method of claim 1 or 2, wherein the used ammonium ion is 1 to 30 mass % in nitrogen with respect to the synthetic mica powder.

4. The method of any of claims 1 to 3, wherein the organic surface treatment agent is an organosilicone, a silane coupling agent, a titanate coupling agent, an aluminate coupling agent, or a perfluoroalkylalkoxysilane.

5. The method of any of claims 1 to 4, wherein the baking temperature is 100 to 260 °C.

6. The method of any of claims 1 to 5, wherein the organic surface treatment agent is an organosilicone and the baking temperature is 170 to 260 °C.

7. The method of any of claims 1 to 6, wherein the organic surface treatment agent contains an organosilicone having Si-H groups.

8. The method of claim 6 or 7, wherein the mole ratio of Si(R)₂O unit: SiH(R)O unit, wherein respective Rs are either identical or different monovalent hydrocarbon groups, in the organic surface treatment agent is within the range of 1:0.05 to 1:0.30.

9. The method of claim 8, wherein R is a methyl group.

10. The method of any of claims 1 to 9, wherein
the synthetic mica powder to be pretreated has the fluoride ion-elution amount of 20 ppm or less by an elution test in hot water at 100 °C for 1 hour and has the t-butanol decomposition rate of less than 20% at 250 °C, and
the pretreated synthetic mica powder has the fluoride ion-elution amount of 20 ppm or less in the hot water elution test at 100 °C for 1 hour and has the t-butanol decomposition rate of 20% or higher at 250 °C.

11. The method of any of claims 1 to 10, wherein 20% or higher of interlayer cations, which has been present on the surface of the synthetic mica powder, are removed in the pretreatment process.

12. An organic-coated synthetic mica powder, said organic-coated synthetic mica powder being obtained by the method of any of claims 1 to 11, wherein the carbon amount in the organic-coated synthetic mica powder is 40 to 60 mass % of the carbon amount in the organic surface treatment agent-attached synthetic mica powder before baking.

13. The organic-coated synthetic mica powder of claim 12, wherein an organosilicone having Si-H groups is used as the organic surface treatment agent, and the hydrogen evolution amount of the organic-coated synthetic mica powder is less than 0.1 mL/g.

14. An organic-coated synthetic mica powder, said organic-coated synthetic mica powder being obtained by attaching an organic surface treatment agent on the surface of synthetic mica powder followed by baking, wherein the carbon amount in the organic-coated synthetic mica powder is 40 to 60 mass % of the carbon amount in the organic surface treatment agent-attached synthetic mica powder before baking.

15. The organic-coated synthetic mica powder of claim 14, wherein the organic surface treatment agent is an organosilicone, a silane coupling agent, a titanate coupling agent, an aluminate coupling agent, or a perfluoroalkylalkoxysilane.

16. The organic-coated synthetic mica powder of claim 14 or 15, wherein the organic surface treatment agent contains an organosilicone having Si-H groups, and the hydrogen generation amount of the organic-coated synthetic mica powder is less than 0.1 mL/g.

17. The organic-coated synthetic mica powder of claim 16, wherein the mole ratio of Si(R)₂O unit: SiH(R)O unit, wherein respective Rs are either identical or different monovalent hydrocarbon groups, in the organic surface treatment agent is whithin the range of 1:0.05 to 1:0.30.

18. The organic-coated synthetic mica powder of claim 17, wherein R is a methyl group.

19. The organic-coated synthetic mica powder of any of claims 12 to 18, wherein the fluoride ion-elution amount thereof by an elution test in hot water at 100 °C for 1 hour is 20 ppm or less.

20. A cosmetic composition, wherein the organic-coated synthetic mica powder of any of claims 12 to 19 is used.

21. A synthetic mica powder to be coated with an organic surface treatment agent, wherein the fluoride ion elution amount thereof by an elution test in hot water at 100°C for 1 hour is 20 ppm or less, and the t-butanol decomposition rate thereof at 250 °C is 20% or higher.

22. The synthetic mica powder to be coated with an organic surface treatment agent of claim 21, wherein 20% or higher of interlayer cations, which has been present on the surface of the synthetic mica powder are removed.
